# EUROPEAN PATENT SPECIFICATION

(11) **EP 3 230 316 B1**
(45) Date of publication and mention of the grant of the patent: **05.01.2022**
(21) Application number: 15819965.3
(22) Date of filing: 10.12.2015
(51) Int. Cl.: C07K 16/22, A61K 39/395, A61K 31/00, A61K 38/17, A61K 31/409, A61K 9/00, A61K 41/00, A61K 31/7105, A61P 27/02

(54) **TREATMENT OF AGE RELATED MACULAR DEGENERATION WITH A SMALL ACTIVE CHOROIDAL NEOVASCULARIZATION LESION**
BEHANDLUNG ALTERSBEDINGTER MAKULADEGENERATION MIT EINER KLEINEN AKTIVEN CHOROIDALEN NEOVASKULARISATIONSLÄSION
TRAITEMENT D'UNE DÉGÉNÉRESCENCE MACULAIRE LIÉE À L'ÂGE ACCOMPAGNÉE D'UNE PETITE LÉSION DE NÉOVASCULARISATION CHOROÏDIENNE ACTIVE

(30) Priority: 11.12.2014 WO PCT/CN2014/093548; 09.09.2015 WO PCT/CN2015/089251
(43) Date of publication of application: 18.10.2017
(60) Divisional application: 21207057.7
(73) Proprietor: Bayer HealthCare LLC, Whippany, NJ 07981 (US)
(72) Inventor: ZEITZ, Oliver, 10249 Berlin (DE); SOWADE, Olaf, 12623 Berlin (DE); WU, Haiyan, Beijing 100096 (CN)
(74) Representative: BIP Patents
(86) International application number: PCT/US2015/065022
(87) International publication number: WO 2016/094673

(56) References cited:
- WO-A2-02/062386
- GUI-SHUANG YING ET AL: "Baseline Predictors for One-Year Visual Outcomes with Ranibizumab or Bevacizumab for Neovascular Age-related Macular Degeneration", OPHTHALMOLOGY., vol. 120, no. 1, 1 January 2013 (2013-01-01), pages 122-129, XP55252719, US ISSN: 0161-6420, DOI: 10.1016/j.ophtha.2012.07.042
- JAVIER ZARRANZ-VENTURA ET AL: "The Neovascular Age-Related Macular Degeneration Database", OPHTHALMOLOGY., vol. 121, no. 10, 1 October 2014 (2014-10-01), pages 1966-1975, XP55252725, US ISSN: 0161-6420, DOI: 10.1016/j.ophtha.2014.04.026
- DAVID M. BROWN ET AL: "Ranibizumab versus Verteporfin for Neovascular Age-Related Macular Degeneration", NEW ENGLAND JOURNAL OF MEDICINE, vol. 355, no. 14, 5 October 2006 (2006-10-05), pages 1432-1444, XP055252324, US ISSN: 0028-4793, DOI: 10.1056/NEJMoa062655
- BROWN D M ET AL: "Ranibizumab versus Verteporfin Photodynamic Therapy for Neovascular Age-Related Macular Degeneration: Two-Year Results of the ANCHOR Study", OPHTHALMOLOGY, J. B. LIPPINCOTT CO., PHILADELPHIA, PA, US, vol. 116, no. 1, 1 January 2009 (2009-01-01), pages 57-65.e5, XP026623775, ISSN: 0161-6420, DOI: 10.1016/J.OPHTHA.2008.10.018 [retrieved on 2009-01-01]
- JEFFREY S. HEIER ET AL: "Intravitreal Aflibercept (VEGF Trap-Eye) in Wet Age-related Macular Degeneration", OPHTHALMOLOGY, vol. 119, no. 12, 1 December 2012 (2012-12-01), pages 2537-2548, XP55138976, ISSN: 0161-6420, DOI: 10.1016/j.ophtha.2012.09.006
- THE PIER STUDY GROUP REGILLO ET AL: "Randomized, Double-Masked, Sham-Controlled Trial of Ranibizumab for Neovascular Age-related Macular Degeneration: PIER Study Year 1", AMERICAN JOURNAL OF OPHTHALMOLOGY, ELSEVIER, AMSTERDAM, NL, vol. 145, no. 2, 25 January 2008 (2008-01-25), pages 239-248.e5, XP022433903, ISSN: 0002-9394, DOI: 10.1016/J.AJO.2007.10.004
- ROSENFELD PHILIP J ET AL: "Ranibizumab for neovascular age-related macular degeneration", NEW ENGLAND JOURNAL OF MEDICINE, MASSACHUSETTS MEDICAL SOCIETY, US, vol. 355, no. 14, 5 October 2006 (2006-10-05), pages 1419-1431, XP002506588, ISSN: 0028-4793, DOI: 10.1056/NEJMOA054481
- Robert P. Finger ET AL: "Predictors of anti-VEGF treatment response in neovascular age-related macular degeneration", SURVEY OF OPHTHALMOLOGY., vol. 59, no. 1, 1 January 2014 (2014-01-01), pages 1-18, XP55495272, XX ISSN: 0039-6257, DOI: 10.1016/j.survophthal.2013.03.009
- ALTAWEEL MICHAEL M ET AL: "Outcomes of Eyes with Lesions Composed of > 50% Blood in the Comparison of Age-Related Macular Degeneration Treatments Trials (CATT)", OPHTHALMOLOGY, vol. 122, no. 2, February 2015 (2015-02), pages 391-398, ISSN: 0161-6420(print)
- OHR MATTHEW ET AL: "Aflibercept in wet age-related macular degeneration: a perspective review.", THERAPEUTIC ADVANCES IN CHRONIC DISEASE JUL 2012, vol. 3, no. 4, July 2012 (2012-07), pages 153-161, ISSN: 2040-6223
- DATABASE BIOSIS [Online] BIOSCIENCES INFORMATION SERVICE, PHILADELPHIA, PA, US; June 2013 (2013-06), ALTAWEEL MICHAEL ET AL: "Clinical Outcomes of Eyes with Neovascular Lesions Composed of > 50% Blood Treated with Anti-VEGF Therapy in the Comparison of AMD Treatments Trials (CATT)", Database accession no. PREV201800956959 & IOVS, vol. 54, no. 15, June 2013 (2013-06), page 4162, ANNUAL MEETING OF THE ASSOCIATION-FOR-RESEARCH-IN-VISION-AND-OPH THALMOLOGY (ARVO); SEATTLE, WA, USA; MAY 05 -09, 2013 ISSN: 0146-0404(print)
- BEAUMONT PAUL E ET AL: "Lesion morphology in age-related macular degeneration and its therapeutic significance", ARCHIVES OF OPHTHALMOLOGY, vol. 124, no. 6, June 2006 (2006-06), pages 807-812, ISSN: 0003-9950

## Description

Age related macular degeneration (AMD) is a medical condition that usually affects older adults and results in a loss of vision in the center of the visual field (the macula) because of damage to the retina. It occurs in "dry" and "wet" forms. In the dry form, cellular debris called drusen accumulates between the retina and the choroid, and the retina can become detached. In the wet form (wAMD), which is more severe, blood vessels grow up from the choroid behind the retina which is also named choroidal neovascularization (CNV). As a result of CNV the retina can also become detached.

The proliferation of abnormal blood vessels in the retina is stimulated by vascular endothelial growth factor (VEGF). Antiangiogenics or anti-VEGF agents can cause regression of the abnormal blood vessels and improve vision when administered intravitreally. Several anti-VEGF drugs have been approved for use in the eye and are described in the following patent applications:

| | |
|---|---|
| Aflibercept (Eylea^{®}) | WO2000/75319 |
| Bevacizumab (Avastin ^{®}) | WO 9845331 |
| Ranibizumab (Lucentis^{®}) | WO9845331 |
| Pegaptanib (Macugen^{®}) | WO9818480 |
| KH-902/conbercept (Langmu^{®}) | WO2007112675 |

Besides anti-VEGF treatment wAMD can be also treated with photodynamic therapy with Visudyne^{®} (V^{®}-PDT), whereby closure of leakage is induced by laser light in combination with verteporfin, an i.v. injectable photosensitizer.

Clinical trials performed with anti-VEGF agents required the inclusion of patients with an active predominantly classic, subfoveal CNVarea that must occupy at least 50% of the total lesion [Rosenfeld et al. N Engl J Med 2006, 355:1419-1431; Brown et al. N Engl J Med 2006, 355:1432-1444; Heier et al. Ophthalmology 2012, 119:2537-2548; Regillo et al. Am J Ophthalmol 2008, 145:239-248; Boyer et al. Ophthalmology 2007, 114:246-252, Schmidt-Erfurth et al. Ophthalmology 2014, 121:193-201; Busbee et al. Ophthalmology 2013, 120:1046-1056]. Hence there is a dearth of information regarding the response of eyes with an active predominantly classic, subfoveal CNVarea that occupies less than 50% of the total lesion to anti-VEGF therapy.

The CATT research group compared the baseline characteristics, treatment frequency, visual acuity (VA), and morphologic outcomes of eyes with >50% of the lesion composed of blood (B50 group) versus all other eyes (Other group) enrolled in the Comparison of Age-Related Macular Degeneration Treatments Trials (CATT). Treatment for the study eye was assigned randomly to either ranibizumab or bevacizumab and to 3 different dosing regimens over a 2-year period. Reading center graders evaluated baseline and follow-up morphology in color fundus photographs, fluorescein angiography (FA), and optical coherence tomography (OCT). Increases in mean visual acuity (VA) were similar in the "B50 group" and the "Other group" at 1 year (+9.3 vs +7.2 letters; P = 0.22) and at 2 years (9.0 vs 6.1 letters; P = 0.17). Mean lesion size in the "B50 group" decreased by 1.2 DA at both 1 and 2 years (primarily owing to resolution of hemorrhage) and increased in the "Other group" by 0.33 DA at 1 year and 0.91 DA at 2 years (P < 0.001). The authors concluded that the "B50 group" had a visual prognosis similar to the "Other group". Lesion size decreased markedly through 2 years. Eyes like those enrolled in CATT with neovascular AMD lesions composed of >50% blood can be managed similarly to those with less or no blood. [Altaweel MM et al. Ophthalmology. 2015 122(2):391-398].

However, the above evaluated subpopulation with >50% of the lesion composed of blood is not equal to the subpopulation of patients with active CNV lesion < 50% of the total lesion size of the study described in this application (example 1).

In accordance with a first aspect, the present invention covers a VEGF antagonist for use in the treatment of wet age related macular degeneration (wAMD) in a patient, wherein it is first established if the size of the active CNV lesion is smaller than 50% of the total lesion size as determined by fluorescence angiography and then the patient is treated with an anti-VEGF treatment in case the size of the active CNV lesion is smaller than 50% of the total lesion size, wherein the VEGF antagonist is aflibercept.

Two subtypes of wAMD can be distinguished: (I) small active CNV lesion - type of wAMD or (II) predominantly active CNV lesion - type of wAMD. The location of the lesion can be subfoveal or juxtafoveal affecting the fovea. The type of the lesion can be of all subtypes including predominantly classic, minimally classic, or occult.

The terminology for the two types of wAMD is preliminary. Alternate terms for the "small active CNV lesion - type of wAMD" may include:
1) "sCNV wAMD"
2) "wAMD with small active CNV"
3) "wAMD with reduced active CNV"
4) "wAMD with less CNV"
5) "Non-CNV related wAMD"
6) "wAMD Type 1"
7) "wAMD Type 2"
8) "wAMD Type X", where X is any number, letter or combination of both.

In the under 1 - 8 listed alternative terms for "reduced active CNV - type of wAMD" alternative terms for "wAMD" can be:
a. "wet AMD"
b. "neovascular AMD"
c. "nAMD"
d. "exudative AMD"
e. "eAMD"

Alternate terms for the "predominantly active CNV lesion - type of wAMD" may include:
1) "pCNV wAMD"
2) "wAMD with predominant active CNV"
3) "wAMD with active CNV"
4) "wAMD with large active CNV"
5) "CNV related wAMD"
6) "wAMD Type 1"
7) "wAMD Type 2"
8) "wAMD Type X", where X is any number, letter or combination of both.

In the under 1 - 8 listed alternative terms for "reduced active CNV - type of wAMD" alternative terms for "wAMD" can be
a. "wet AMD"
b. "neovascular AMD"
c. "nAMD"
d. "exudative AMD"
e. "eAMD"

In the following, the terms "sCNV wAMD" and "pCNV wAMD" will be used.

While the presence of active CNV lesion and thereby the diagnosis of wAMD is usually confirmed by fluorescence angiography (FA), the two wAMD types can be differentiated as follows:
1) "sCNV wAMD" is characterized by an active CNV lesion that occupies less than 50% of the total lesion size
2) "pCNV wAMD" is characterized by an active CNV lesion that occupies at least 50% of the total lesion size.

The location of the lesion can be subfoveal or juxtafoveal affecting the fovea. The type of the lesion can be of all subtypes including predominantly classic, minimally classic or occult.

The size of the active CNV lesion as well as the total lesion size is determined using Fluorescence Angiography (FA) as described in the MPS protocol [Macular Photocoagulation Study Group, Arch Ophthalmol 1991, 109:1242-1257].

With the invention, it is shown that lesions with small active portion of the CNV lesion (< 50% of total lesion size; "sCNV wAMD") respond well to treatment with anti-VEGF treatment, namely aflibercept. This conclusion is based on an observation made in a clinical trial with patients with "sCNV wAMD" and "pCNV wAMD" which were treated with intravitreal injection of aflibercept Surprisingly, the response determined by visual acuity of the "sCNV wAMD" patients to the aflibercept treatment was numerically higher to the response of the "pCNV wAMD" patients. This was not expected because it is assumed that lesions with a large active portion of the CNV lesion are more receptive to the anti-leakage effect of the anti-VEGF treatment than lesions with small active portion of the CNV lesion.

According to the invention, treatments for wAMD can be also used for the treatment of patients with "sCNV wAMD". Such treatment of patients with "sCNV wAMD" may be as follows:
1) Intravitreal anti-VEGF monotherapy similar to the treatment of wAMD, whereas anti-VEGF therapy refers to all approved and non-approved treatments aiming to attenuate free VEGF in the eye. This includes particularly aflibercept, ranibizumab, bevacizumab, KH-902, and pegaptanib, but is not limited to these compounds. Anti-VEGF treatment may be applied according to the following treatment schedules:
   a. Three monthly intravitreal injections or three intravitreal injections each 4 weeks apart followed by dosing every other month or every 4 weeks with or without the option to extend the treatment interval further during the later treatment phase.
   b. Treatment until visual acuity and/or retinal morphology (e.g. as assessed by OCT, Fluoresceine Angiography, Indocyanine Angiography, Funduscopy, etc.) stabilizes, followed by discontinuation of treatment. Re-initiation of treatment upon deterioration of visual acuity and/or retinal morphology.
   c. Any as needed (pro-re-nata - "PRN") regimen
   d. Any Treat&Extend regimen
   e. Any other treatment regimen that is or has been used for treatment of wAMD
2) Therapy with one or more of the following treatments. If more than one treatment is used, they may be used at the same time or sequentially.
   a. Anti-VEGF treatment as described under 1)
   b. Single or repeated applications of photodynamic therapy with Visudyne^{®} (V^{®}-PDT)
   c. Single or repeated applications of steroids (all available local or systemic application routes) including slow-release or depot formulations (e.g. Ozurdex, triamcinolone, dexamethasone, Iluvien, etc.)
   d. Radiation therapy
   e. Thermal laser therapy including sub-threshold treatments
   f. Surgical therapy
   g. Pharmacological vitreolysis (e.g. with Jetria or other approved or non-approved drugs)
   h. Systemically or locally applied inhibitors of tyrosine kinases
   i. Systemically or locally applied inhibitors of the VEGF receptor

### Example 1:

A total of 304 Chinese subjects with age-related neovascular or wet age-related macular degeneration were enrolled in a randomized, double-blind clinical study to assess the efficacy of intravitreal (IVT) administrated aflibercept compared with V^{®}-PDT on the mean change in BCVA (Best corrected visual acuity) from baseline to Week 28. BCVA of the study eye was assessed according to the standard procedures developed for the ETDRS (Early Treatment Diabetic Retinopathy Study) adapted for Age Related Eye Disease Study (AREDS). The key inclusion criteria were as follows:
- Signed and dated written ICF.
- Men and women ≥50 years of age.
- Active predominantly classic, subfoveal choroidal neovascularization (CNV) lesions secondary to AMD, including juxtafoveal lesions that affect the fovea, as evidenced by fluorescein angiography (FA), in the study eye.
- The area of the CNV had to occupy at least 50% of the total lesion.
- ETDRS best corrected visual acuity (BCVA) of 73 to 25 letters in the study eye (Snellen activity equivalent of 20/40 to 20/320 in the study eye).

The following key exclusion criteria applied for the study eye:
- Total lesion size is greater than 12 disc areas (30.5 mm², including blood, scars and neovascularization), as assessed by FA
- Sub-retinal hemorrhages that is ≥50% of the total lesion area, or if the blood is under the fovea and is 1 or more disc areas in size. (If the blood is under the fovea, then the fovea must be surrounded by 270 degrees by visible CNV.)
- Presence of CNV with an origin other than wAMD. History or clinical evidence of diabetic retinopathy, diabetic macular edema or any retinal vascular disease other than wAMD. Particular attention should be made to exclude subjects with polypoidal choroidal vasculopathy (PCV).
- Presence of scar, fibrosis, or atrophy involving the center of the fovea that indicates substantial irreversible vision loss.
- Presence of retinal pigment epithelial tears or rips involving the macula.

Eligible patients were randomized 3:1 to receive aflibercept (VTE) 2Q8 or V^{®}-PDT (228 VTE+76 PDT). 194 patients with active CNV lesions >= 50% (147 VTE2Q8 + 47 PDT) and 106 patients with active CNV lesions < 50% (78 VTE2Q8 + 28 PDT) were included. The lesion size was determined by a central reading center based on the MPS protocol [Macular Photocoagulation Study Group, Arch Ophthalmol 1991, 109:1242-1257]. The active CNV size, the area of CNV (mm²) as well as the total lesion size was measured using the FA. The central retinal thickness was determined by optical coherence tomography. In the VTE2Q8 group patients were treated with 2 mg (0.05 mL) aflibercept administered intravitreally at baseline, week 4, 8, 16, 24, 32, 40 and 48. In the PDT group V^{®}-PDT was performed at baseline and potential PDT retreatment according to the guidelines for the use of PDT treatment in wAMD [Verteporfin Roundtable Participants, Retina. 2005; 25(2):119-34] were performed at week 12 and 24. At Week 28, after assessment of the primary and secondary endpoints, subjects in the PDT→VEGF Trap-Eye group received an IVT injection of 2.0 mg VEGF Trap-Eye, followed by additional 2.0 mg VEGF Trap-Eye injections at Weeks 32, 36, 40, and 48.

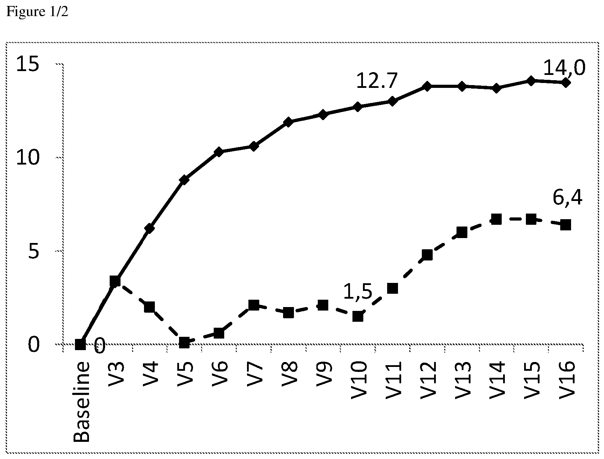

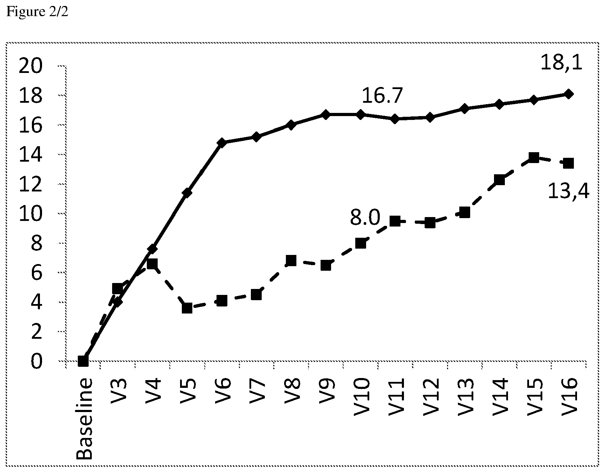

Intravitreal injections of 2 mg aflibercept was superior to V^{®}-PDT with a mean change from baseline BCVA letter score at week 28 of 14.0 (-29 to 59) VTE2Q8 group versus 3.9 (-36 to 43) PDT group (P<0.0001) in the whole study population irrespective of the active CNV lesion size. Intravitreal injection of 2 mg aflibercept provided an effective treatment for patients with an active CNV lesion < 50% of total lesion size (mean change of BCVA from baseline at week 28: 16.7 (-21 to 59) see figure 2/2) which was comparable to the treatment outcome of patients with an active CNV lesion >= 50% of total lesion size (mean change of BCVA from baseline at week 28:12.7 (-29 to 40) see figure 1/2). V^{®}-PDT treatment effect is numerically higher in patients with an active CNV lesion < 50% of total lesion size (mean change of BCVA from baseline at week 28: 8.0 (-18 to 43), see figure 2/2) than in patients with an active CNV lesion >= 50% of total lesion size (mean change of BCVA from baseline at week 28: 1.5 (-36 to 27) see figure 1/2).

In general, for most of the other efficacy parameters a more favorable outcome in patients with an active CNV lesion <50% of the total lesion size compared to those with an active CNV lesion >=50% of the total lesion size was observed for patients both treated with VTE2Q8 and V^{®}-PDT (table 1).

**Table 1: Efficacy outcome for VTE2Q8 and V^{®}-PDT in patients with active CNV lesion size <50% and >=50% of total lesion size at week 28**

| | VTE2Q8 | | V^{®}-PDT | |
|---|---|---|---|---|
| Efficacy parameter (unit) | Active CNV lesion <50%* | Active CNV lesion >=50%* | Active CNV lesion <50%* | Active CNV lesion >=50%* |
| Mean change of BCVA (letters) | 16.7 | 12.7 | 8.0 | 1.5 |
| Proportion of patients who maintained vision (letter loss <15 letters) (%) | 97.4 | 99.3 | 92.9 | 91.7 |
| Proportion of patients who gained 5 or more letters (%) | 85.9 | 77.6 | 50.0 | 41.7 |
| Proportion of patients who gained 10 or more letters (%) | 73.1 | 66.7 | 32.1 | 27.1 |
| Proportion of patients who gained 15 or more letters (%) | 57.7 | 45.6 | 25.0 | 12.5 |
| Proportion of patients who lost 5 or | 5.1 | 6.8 | 17.9 | 31.3 |
| more letters (%) | | | | |
| Proportion of patients who lost 10 or more letters (%) | 3.8 | 2.7 | 14.3 | 18.8 |
| Proportion of patients who lost 15 or more letters (%) | 2.6 | 0.7 | 7.1 | 8.3 |
| Mean change in central retinal thickness (um) | -180.6 | -180.4 | - 109.5 | -91.6 |
| Mean change in CNV lesion size (mm²) | -0.688 | -1.009 | -0.286 | -0.201 |

| | | | | |
|---|---|---|---|---|
| * Active CNV lesion of total lesion size | | | | |

**Table 2: Efficacy outcome for VTE2Q8 and V^{®}-PDT in patients with active CNV lesion size <50% and >=50% of total lesion size at week 52**

| | VTE2Q8 | | V^{®}-PDT | |
|---|---|---|---|---|
| Efficacy parameter (unit) | Active CNV lesion <50%* | Active CNV lesion >=50%* | Active CNV lesion <50%* | Active CNV lesion >=50%* |
| Mean change of BCVA (letters) | 18.1 | 14.0 | 13.4 | 6.4 |
| Proportion of patients who maintained vision (letter loss <15 letters) (%) | 96.2 | 98.0 | 96.4 | 87.5 |
| Proportion of patients who gained 5 or more letters (%) | 88.5 | 78.9 | 67.9 | 60.4 |
| Proportion of patients who gained 10 or more letters (%) | 78.2 | 67.3 | 57.1 | 50.0 |
| Proportion of patients who gained 15 or more letters (%) | 69.2 | 53.1 | 46.4 | 39.6 |
| Proportion of patients who lost 5 or more letters (%) | 6.4 | 8.2 | 21.4 | 29.2 |
| Proportion of patients who lost 10 or more letters (%) | 3.8 | 4.1 | 10.7 | 16.7 |
| Proportion of patients who lost 15 or more letters (%) | 3.8 | 2.0 | 3.6 | 12.5 |
| Mean change in central retinal thickness (um) | -185.5 | -192.1 | -176.0 | -166.6 |
| Mean change in CNV lesion size (mm²) | -0.688 | -1.173 | -0.286 | -1.213 |

| | | | | |
|---|---|---|---|---|
| * Active CNV lesion of total lesion size | | | | |

### Description of the figures:

**Figure 1****/****2****: Mean change from baseline in ETDRS BCVA letter score by visit in subjects with an active CNV lesion ≥ 50% of total lesion size at baseline. The mean change in BCVA score (no. of** letters) as measured by ETDRS from baseline at week 1 (V3) week 4 (V4), week 8 (V5), week 12 (V6), week 16 (V7), week 20 (V8), week 24 (V9), week 28 (V10), Week 32 (Visit 11), Week 36 (Visit 12), Week 40 (Visit 13), Week 44 (visit 14), Week 28 (Visit 15), Week 52 (Visit 16) is shown for the VTE2Q8 group (solid line with diamonds) and the PDT->VTE group (dashed line with squares). At week 28 (V10) the mean change in BCVA score from baseline is 12.7 for the VTE2Q8 group and 1.5 for the PDT->VTE group. At week 52, the mean change in BCVA score from baseline is 14.0 for the VTE2Q8 group and 6.4 for the PDT->VTE group.

**Figure 2/2****: Mean change from baseline in ETDRS BCVA letter score by visit in subjects with an active CNV lesions < 50% of total lesion size at baseline.** The mean change in BCVA score (no. of letters) as measured by ETDRS from baseline at week 1 (V3) week 4 (V4), week 8 (V5), week 12 (V6), week 16 (V7), week 20 (V8), week 24 (V9), week 28 (V10), Week 32 (Visit 11), Week 36 (Visit 12), Week 40 (Visit 13), Week 44 (visit 14), Week 28 (Visit 15), Week 52 (Visit 16) is shown for the VTE2Q8 group (solid line with diamonds) and the PD->VTET group (dashed line with squares). At week 28 (V10) the mean change in BCVA score from baseline is 16.7 for the VTE2Q8 group and 8.0 for the PDT->VTE group. At week 52 (V10) the mean change in BCVA score from baseline is 18.1 for the VTE2Q8 group and 13.4 for the PDT->VTE group.

## Claims

1. A VEGF antagonist for use in the treatment of wet age related macular degeneration (wAMD) in a patient, wherein it is first established if the size of the active CNV lesion is smaller than 50% of the total lesion size as determined by fluorescein angiography and then the patient is treated with an anti-VEGF treatment in case the size of the active CNV lesion is smaller than 50% of the total lesion size, wherein the VEGF antagonist is aflibercept.

2. A VEGF antagonist for use in the treatment of wAMD according to claim 1, wherein the initial anti-VEGF treatment comprises of at least 3 injections of the VEGF antagonist each 4 weeks apart.

## Patentansprüche

1. VEGF-Antagonist zur Verwendung bei der Behandlung von feuchter altersbedingter Makuladegeneration (wAMD) bei einem Patienten, wobei zuerst festgestellt wird, ob die Größe der aktiven CNV-Läsion kleiner als 50% der Gesamtläsionsgröße gemäß Bestimmung mittels Fluoreszenzangiografie ist, wonach der Patient mit einer anti-VEGF-Behandlung behandelt wird, falls die Größe der aktiven CNV-Läsion kleiner als 50% der Gesamtläsionsgröße ist, wobei der VEGF-Antagonist Aflibercept ist.

2. VEGF-Antagonist zur Verwendung bei der Behandlung von wAMD nach Anspruch 1, wobei die anfängliche anti-VEGF-Behandlung mindestens 3 Injektionen des VEGF-Antagonisten jeweils im Abstand von 4 Wochen umfasst.

## Revendications

1. Antagoniste de VEGF pour utilisation dans le traitement de la dégénérescence maculaire liée à l'âge humide (DMLAh) chez un patient, où il est établi dans un premier temps si la taille de la lésion du SNC active est inférieure à 50 % de la taille totale de lésion comme déterminé par angiographie à la fluorescéine, puis le patient est traité avec un traitement anti-VEGF dans le cas où la taille de la lésion du SNC active est inférieure à 50 % de la taille totale de lésion, où l'antagoniste de VEGF est l'aflibercept.

2. Antagoniste de VEGF pour utilisation dans le traitement de la DMLAh selon la revendication 1, où le traitement anti-VEGF initial comprend au moins 3 injections de l'antagoniste de VEGF chacune à 4 semaines d'intervalle.
